(19) Europäisches Patentamt
European Patent Office
Office européen des brevets

(11) Veröffentlichungsnummer : **0 217 038**
**B1**

(12) # EUROPÄISCHE PATENTSCHRIFT

(45) Veröffentlichungstag der Patentschrift : **07.06.89**

(51) Int. Cl.⁴ : **C 07 D417/12**, A 01 N 43/72

(21) Anmeldenummer : **86110231.7**

(22) Anmeldetag : **24.07.86**

(54) **Benzo-[b]-1,4,5,7-dithiadiazepin-1,1,4,4-tetraoxydderivate.**

(30) Priorität : **06.08.85 DE 3528099**

(43) Veröffentlichungstag der Anmeldung : **08.04.87 Patentblatt 87/15**

(45) Bekanntmachung des Hinweises auf die Patenterteilung : **07.06.89 Patentblatt 89/23**

(84) Benannte Vertragsstaaten : **AT BE CH DE FR GB IT LI NL**

(56) Entgegenhaltungen :
**EP-A- 0 173 314**
**CHEMICAL ABSTRACTS, Band 87, nr. 9, 29. August 1977, Columbus, Ohio, USA, M. VINCENT; G. REMOND; J.P. VOLLAND "Condensation of bis(chlorosulfonyl)methane with some 1,2- and 1,3-diamines. Studies on the resulting heterocycles", p. 549, Zusammenfassung Nr. 68 313f**

(73) Patentinhaber : **BAYER AG**
**D-5090 Leverkusen 1 Bayerwerk (DE)**

(72) Erfinder : **Fest, Christa, Dr.**
**Im Johannistal 20**
**D-5600 Wuppertal 1 (DE)**
Erfinder : **Kirsten, Rolf, Dr.**
**Carl-Langhans-Strasse 27**
**D-4019 Monheim (DE)**
Erfinder : **Kluth, Joachim, Dr.**
**Kurt-Schumacher-Strasse 9**
**D-4018 Langenfeld (DE)**
Erfinder : **Müller, Klaus-Helmut, Dr.**
**Bockhackstrasse 55**
**D-4000 Düsseldorf 13 (DE)**
Erfinder : **Pfister, Theodor, Dr.**
**Lichtenberger Strasse 30**
**D-4019 Monheim (DE)**
Erfinder : **Priesnitz, Uwe, Dr.**
**Severinstrasse 58**
**D-5650 Solingen (DE)**
Erfinder : **Riebel, Hans-Jochem, Dr.**
**In der Beek 92**
**D-5600 Wuüppertal 1 (DE)**
Erfinder : **Roy, Wolfgang, Dr.**
**Walter-Kolb-Strasse 47**
**D-4018 Langenfeld (DE)**
Erfinder : **Santel, Hans-Joachim, Dr.**
**Grünstrasse 9a**
**D-5090 Leverkusen 1 (DE)**
Erfinder : **Schmidt, Robert R., Dr.**
**Im Waldwinkel 110**
**D-5060 Bergisch Gladbach (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft neuartige Benzo-[b]-1,4,5,7-dithiadiazepin-1,1,4,4-tetraoxid-Derivate (zur Vereinfachung nachfolgend als « Benzodisultame » bezeichnet), welche eine neue Substanzklasse bilden, ein erfinderisches Verfahren zu deren Herstellung und deren Verwendung als Herbizide.

Benzodisultame sind bisher nicht aus der vorveröffentlichten Literatur bekannt. Die Verwendung ähnlicher Verbindungen als Herbizide ist bisher ebenfalls nicht bekannt. Erstmals sind Benzodisultame, die in 6-Stellung durch Pyrimidinylamino-Gruppen substituiert sind, sowie deren Verwendung als Herbizide in einer eigenen älteren, aber nicht vorveröffentlichten Patentanmeldung (vgl. EP-A-173 314) beschrieben worden.

Es wurden nun neue, in 6-Stellung durch Triazinylamino-Gruppen substituierte Benzodisultame der allgemeinen Formel (I)

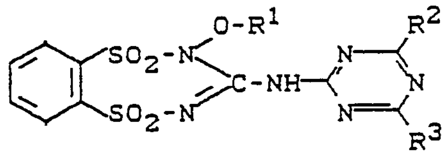

(I)

gefunden, in welcher

$R^1$ für $C_1$-$C_{12}$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkyl-amino-carbonyl oder Di-($C_1$-$C_4$-alkyl)-amino-carbonyl substituiert ist], für $C_3$-$C_6$-Alkenyl [welches gegebenenfalls durch Fluor, Chlor oder Brom substituiert ist], $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxycarbonyl substituiert ist] oder für Phenyl [welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist] steht, in welcher weiter

$R^2$ für Wasserstoff, Fluor, Chlor, Hydroxy, Cyclopropyl, Methyl [welches gegebenenfalls durch Fluor und/oder Chlor substituiert ist], für $C_1$-$C_3$-Alkoxy [welches gegebenenfalls durch Fluor und/oder Chlor substituiert ist], für $C_1$-$C_3$-Alkylthio [welches gegebenenfalls durch Fluor und/oder Chlor substituiert ist], Amino, für $C_1$-$C_3$-Alkyl- oder Di-($C_1$-$C_3$-alkyl)-amino [welche gegebenenfalls durch Fluor substituiert sind] steht und

$R^3$ für Wasserstoff, Fluor, Chlor, Hydroxy, Cyclopropyl, Methyl [welches gegebenenfalls durch Fluor und/oder Chlor substituiert ist], für $C_1$-$C_3$-Alkoxy [welches gegebenenfalls durch Fluor und/oder Chlor substituiert ist], für $C_1$-$C_3$-Alkylthio [welches gegebenenfalls durch Fluor und/oder Chlor substituiert ist], Amino, für $C_1$-$C_3$-Alkyl- oder Di-($C_1$-$C_3$-alkyl)-amino [welche gegebenenfalls durch Fluor substituiert sind] steht.

Man erhält die neuen Verbindungen der Formel (I), wenn man Benzol-1,2-disulfonsäure-dichlorid der Formel (II)

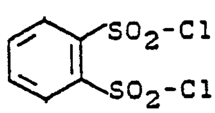

(II)

mit Oxyguanidin-Derivaten der Formel (III)

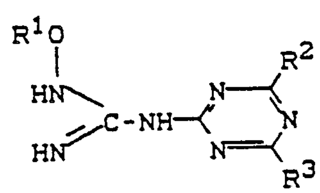

(III)

in welcher $R^1$, $R^2$ und $R^3$ die oben angegebenen Bedeutungen haben, in Gegenwart von Säureakzeptoren und gegebenenfalls in Gegenwart von Verdünnungsmitteln umsetzt.

Die neuen Benzodisultame der Formel (I) zeichnen sich durch starke herbizide Wirksamkeit aus.

Überraschenderweise zeigen die neuen Verbindungen der Formel (I) erheblich stärkere herbizide Wirkung als viele bekannte chemische Verbindungen gleicher Wirkungsrichtung.

Gegenstand der Erfindung sind vorzugsweise Verbindungen der Formel (I), in welcher

$R^1$ für $C_1$-$C_8$-Alkyl [welches gegebenenfalls durch Fluor oder Chlor substituiert ist], $C_3$-$C_4$-Alkenyl,

$C_1$-$C_2$-Alkoxy-carbonylmethyl, Phenyl, Phenylethyl oder Benzyl [welches gegebenenfalls durch Fluor, Chlor, Nitro, Cyano, Methyl, Methoxy oder Methoxy-carbonyl substituiert ist] steht,

$R^2$ für Wasserstoff, Chlor, Methyl, Methoxy, Ethoxy, Methylthio, Ethylthio, Methylamino, Ethylamino, Dimethylamino oder Diethylamino steht und

$R^3$ für Wasserstoff, Chlor, Methyl, Methoxy, Ethoxy, Methylthio, Ethylthio, Methylamino, Ethylamino, Dimethylamino oder Diethylamino steht.

Verwendet man beim erfindungsgemäßen Verfahren beispielsweise N'-(4,6-Dimethyl-1,3,5-triazin-2-yl)-N''-allyloxy-guanidin und Benzol-1,2-disulfonsäure-dichlorid als Ausgangsstoffe, so kann der Reaktionsablauf durch folgendes Formelschema skizziert werden :

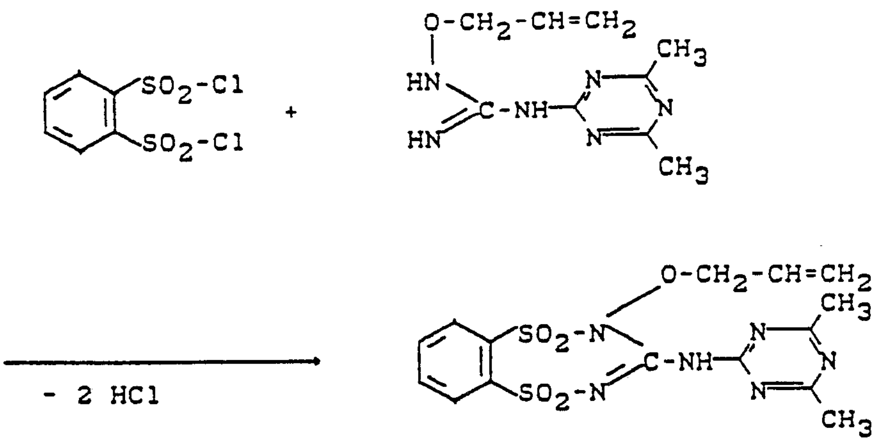


Das beim erfindungsgemäßen Verfahren als Ausgangsstoff zu verwendende Benzol-1,2-disulfonsäure-dichlorid der Formel (II) ist bereits bekannt (vergl. J. Org. Chem. 31 (1966), 3 289-3 292).

Die beim erfindungsgemäßen Verfahren weiter als Ausgangsstoffe zu verwendenden Oxyguanidin-Derivate sind durch die Formel (III) allgemein definiert. In Formel (III) haben $R^1$, $R^2$ und $R^3$ vorzugsweise die gleichen Bedeutungen, wie sie oben im Rahmen der Substituentendefinition der Formel (I) vorzugsweise angegeben sind.

Als Ausgangsstoffe der Formel (III) seien beispielsweise genannt :

N'-(4,6-Dimethyl-s-triazin-2-yl)-, N'-(4-Methoxy-6-methyl-s-triazin-2-yl)-, N'-(4-Ethoxy-6-methyl-s-triazin-2-yl)-, N'-(4,6-Dimethoxy-s-triazin-2-yl)-, N'-(4,6-Diethoxy-s-triazin-2-yl)-, N'-(4-Ethoxy-6-methoxy-s-triazin-2-yl)-, N'-(4-Methyl-6-methylthio-s-triazin-2-yl)-, N'-(4-Ethylthio-6-methyl-s-triazin-2-yl)-, N'(4-Methoxy-6-methylthio-s-triazin-2-yl)-, N'-(4-Ethoxy-6-methylthio-s-triazin-2-yl)-, N'-(4-Ethylthio-6-methoxy-s-triazin-2-yl)-, N'-(4-Ethoxy-6-ethylthio-s-triazin-2-yl)-, N'-(4,6-Bis-methylthio-s-triazin-2-yl)-, N'-4,6-(Bis-ethylthio-s-triazin-2-yl)-, N'-(4-Methyl-6-methyl-amino-s-triazin-2-yl)-, N'-(4-Ethylamino-6-methyl-s-triazin-2-yl)-, N'-(4-Dimethylamino-6-methyl-s-triazin-2-yl)-, N'-(4-Diethylamino-6-methyl-s-triazin-2-yl)-, N'-(4-Methoxy-6-methylamino-s-triazin-2-yl)-, N'-(4-Ethylamino-6-methoxy-s-triazin-2-yl)-, N'-(4-Dimethyl-amino-6-methoxy-s-triazin-2-yl)-, N'-(4-Diethylamino-6-methoxy-s-triazin-2-yl)-, N'-(4-Ethoxy-6-methylamino-s-triazin-2-yl)-, N'-(4-Ethoxy-6-ethylamino-s-triazin-2-yl)-, N'-(4-Dimethylamino-6-ethoxy-s-triazin-2-yl)-, N'-(4-Diethylamino-6-ethoxy-s-triazin-2-yl)-, N'-(4-Methylamino-6-methylthio-s-triazin-2-yl)-, N'-(4-Ethylamino-6-methylthio-s-triazin-2-yl)-, N'-(4-Dimethylamino-6-methylthio-s-triazin-2-yl)-, N'-(4-Diethylamino-6-methylthio-s-triazin-2-yl)-, N'-(4-Ethylthio-6-methylamino-s-triazin-2-yl)-, N'-(4-Dimethylamino-6-ethylthio-s-triazin-2-yl)- und N'-(4-Diethylamino-6-ethylthio-s-triazin-2-yl)-N''-methoxy-guanidin, —N''-ethoxy-guanidin, —N''-propoxy-guanidin, —N''-isopropoxy-guanidin, —N''-butoxy-guanidin, —N''-isobutoxy-guanidin, —N''-sec.-butoxy-guanidin, —N''-pentoxy-guanidin, —N''-isopentoxy-guanidin, —N''-hexyloxy-guanidin, —N''-octyloxy-guanidin, —N''-allyloxy-guanidin, —N''-(2-chlor-ethoxy)-guanidin, —N''-(2-fluorethoxy)-guanidin, —N''-(2-chlor-propoxy)-guanidin, —N''-(2-fluor-propoxy)-guanidin, —N''-(3-chlor-propoxy)-guanidin, —N''-(4-chlor-butoxy)-guanidin, —N''-methoxycarbonylmethoxy-guanidin, —N''-ethoxycarbonylmethoxy-guanidin, —N''-(1-methoxy-carbonylethoxy)-guanidin, —N''-(1-ethoxycarbonyl-ethoxy)-guanidin, —N''-dimethylamino-carbonylmethoxy-guanidin, —N''-(2-phenyl-ethoxy)-guanidin, —N''-phenoxy-guanidin, —N''-(4-methylbenzyloxy)-guanidin, —N''-(4-fluorbenzyloxy)-guanidin, —N''-(4-chlor-benzyloxy)-guanidin, —N''-(4-nitro-benzyloxy)-guanidin, —N''-(2,6-dichlor-benzyloxy)-guanidin, —N''-(4-methoxycarbonylbenzyloxy)-guanidin und —N''-(4-ethoxycarbonyl-benzyloxy)-guanidin.

Die Ausgangsstoffe der Formel (III) sind zum Teil bekannt (vergl. EP-A 121 082).

Die Cyanaminotriazin-Derivate der Formel (IV) sind bekannt (vergl. DE-OS 3 334 455).

Man erhält die Verbindungen der Formel (IV) im wesentlichen nach folgenden Synthesewegen :

(a[1]) Durch Umsetzungen von Alkalimetall- oder Erdalkalimetall-Salzen von Cyanamid — wie z. B. Natriumcyanamid oder Calciumcyanamid — mit Chlortriazinen der Formel (VI)

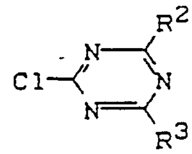

(VI)

in welcher $R^2$ und $R^3$ die oben angegebenen Bedeutungen haben, gegebenenfalls in Gegenwart von Säureakzeptoren und gegebenenfalls in Gegenwart von Verdünnungsmitteln, wie z. B. Ethanol, Aceton, Acetonitril, Dimethylformamid, Dimethylsulfoxid oder Wasser, bei Temperaturen zwischen — 10 °C und 100 °C. Nach Einengen und Auflösen des Rückstandes in Wasser können die Cyanaminotriazin-Derivate der Formel (IV) durch Ansäuern, z. B. mit Salzsäure, ausgefällt und durch Absaugen isoliert werden.

Man erhält die Verbindungen der Formel (III) wenn man Cyanaminotriazin-Derivate der Formel (IV)

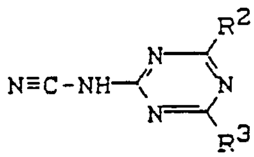

(IV)

in welcher $R^2$ und $R^3$ die oben angegebenen Bedeutungen haben, mit Hydroxylamin-Derivaten der Formel (V)

$$H_2N—OR^1 \qquad (V)$$

in welcher $R^1$ die oben angegebene Bedeutung hat, bzw. deren Hydrochloriden, gegebenenfalls in Gegenwart von Verdünnungsmitteln, wie z. B. Ethanol, Propanol oder Butanol, bei Temperaturen zwischen — 20 °C und 150 °C umsetzt und gegebenenfalls die Umsetzungsprodukte mit Säureakzeptoren, wie z. B. Ammoniak, Kaliumcarbonat oder Natriumhydroxid, behandelt.

Die als Ausgangsstoffe für das vorausgehend unter ($a^1$) beschriebene Herstellungsverfahren für die Cyanaminotriazin-Derivate der Formel (IV) zu verwendenden Chlortriazine der Formel (VI) sind bekannt und/oder können nach an sich bekannten Verfahren hergestellt werden (vgl. US-PS 3 154 547 und US-PS 4 160 037).

In einem weiteren Verfahren erhält man Cyanaminotriazin-Derivate der Formel (IV), wenn man

($a^2$) Dichlortriazine der Formel (VIa)

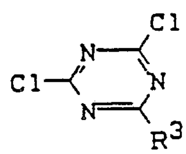

(VIa)

in welcher $R^3$ die oben angegebene Bedeutung hat, mit Alkalimetallsalzen oder Erdalkalimetallsalzen von Cyanamid, wie z. B. mit Dinatriumcyanamid, in Gegenwart eines Verdünnungsmittels, wie z. B. Wasser, bei Temperaturen zwischen — 10 °C und + 50 °C umsetzt und das hierbei anfallende Metallsalz des Monochlor-cyanaminotriazins der Formel (IVa)

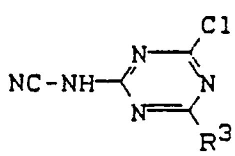

(IVa)

in welcher $R^3$ die oben angegebene Bedeutung hat, mit einer Säure, wie z. B. Salzsäure, in das freie Monochlor-cyanaminotriazin der Formel (IVa) umwandelt.

Aus den Monochlor-cyanaminotriazinen der Formel (IVa) können durch Umsetzung mit Alkoholen bzw. Alkanthiolen oder mit Alkalimetallsalzen dieser Verbindungen bzw. mit Monoalkyl- oder Dialkylaminen die entsprechenden Cyanaminotriazine der Formel (VI), in welcher $R^2$ für Alkoxy, Alkylthio, Alkylamino oder Dialkylamino steht, hergestellt werden.

Die weiter als Ausgangsstoffe zu verwendenden Hydroxylamin-Derivate der Formel (V) sind ebenfalls bekannt und können nach an sich bekannten Verfahren hergestellt werden (vergl. Chem. Pharm. Bull. 15 (1967), 345 ; Bull. Soc. Chim. France 1958, 664 ; Synthesis 1976, 682 ; J. Chem. Soc. 1930, 228 und Helv. Chim. Acta 45 (1962), 1387).

Das erfindungsgemäße Verfahren zur Herstellung der neuen Verbindungen der Formel (I) wird

vorzugsweise unter Verwendung von Verdünnungsmitteln durchgeführt. Als solche kommen praktisch alle inerten organischen, vorzugsweise jedoch aprotisch polare Solventien in Betracht. Hierzu gehören gegebenenfalls halogenierte Kohlenwasserstoffe, wie z. B. Methylenchlorid, Chloroform, Toluol und Chlorbenzol, Nitrile, wie z. B. Acetonitril und Propionsäurenitril, Dimethylformamid, Dimethylacetamid, Dimethylsulfoxid, Sulfolan, Hexamethylphosphorsäuretriamid, 1,2-Dimethoxyethan, Pyridin und 2-Methyl-5-ethyl-pyridin.

Als Säureakzeptoren können beim erfindungsgemäßen Verfahren praktisch alle üblicherweise verwendeten Säurebindemittel eingesetzt werden. Hierzu gehören insbesondere Alkalimetall- und Erdalkalimetall-hydride, metallorganische Verbindungen, wie Butyllithium, ferner aliphatische, aromatische oder heterocyclische Amine, wie Trimethylamin, Triethylamin, N,N-Dimethylanilin, N,N-Dimethylbenzylamin, Diazabicyclooctan (DABCO), Diazabicycloundecen (DBU), Pyridin, 2-Methyl-5-ethyl-pyridin und 4-Dimethylamino-pyridin.

Die Reaktionstemperaturen können beim erfindungsgemäßen Verfahren innerhalb eines größeren Bereichs variiert werden. Im allgemeinen arbeitet man zwischen — 80 °C und + 100 °C, vorzugsweise zwischen — 40 °C und + 50 °C. Das erfindungsgemäße Verfahren wird im allgemeinen bei Normaldruck durchgeführt.

Zur Durchführung des erfindungsgemäßen Verfahrens setzt man je Mol Oxyguanidin-Derivat der Formel (III) im allgemeinen zwischen 1,0 und 1,5 Mol, vorzugsweise zwischen 1,0 und 1,2 Mol Benzol-1,2-disulfonsäure-dichlorid der Formel (II) ein. Die Reaktionskomponenten werden gewöhnlich bei Raumtemperatur oder unter Außenkühlung zusammengegeben und das Reaktionsgemisch wird bis zum Reaktionsende gerührt.

Die Aufarbeitung und Isolierung der neuen Verbindungen der Formel (I) kann nach üblichen Methoden durchgeführt werden. Beispielsweise wird das Reaktionsgemisch — gegebenenfalls nach Verdünnen mit einem mit Wasser praktisch nicht mischbaren Lösungsmittel, wie z. B. Methylenchlorid — mit verdünnter Salzsäure und mit Wasser gewaschen, getrocknet, filtriert und eingeengt. Das im Rückstand verbliebene Produkt der Formel (I) wird durch Verreiben mit einem geeigneten organischen Lösungsmittel, wie z. B. Ethanol, zur Kristallisation gebracht und durch Absaugen isoliert.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind.

Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden :

Dikotyle Unkräuter der Gattungen : Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen : Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen : Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen : Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe, Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %. Die Formulierungen können einen Gehalt an mindestens einem erfindungsgemäßen Wirkstoff der Formel (I) haben.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z. B. N-(2-Benzthiazolyl)-N,N'-dimethylharnstoff, 3-(3-Chlor-4-methylphenyl)-1,1-dimethylharnstoff, 3-(4-Isopropylphenyl)-1,1-dimethylharnstoff, 4-Amino-6-(1,1-dimethylethyl)-3-methylthio-1,2,4-triazin-5-(4H)-on, 4-Amino-6-(1,1-dimethyl-ethyl)-3-ethylthio-1,2,4-triazin-5-(4H)-on, 1-Amino-6-ethylthio-3-(2,2-dimethylpropyl)-1,3,5-triazin-2,4-(1H, 3H)-dion, 4-Amino-3-methyl-6-phenyl-1,2,4-triazin-5-(4H)-on, 2-Chlor-4-ethylamino-6-isopropylamino-1,3,5-triazin, das R-Enantiomere des 2-[4-(3,5-Dichlor-pyridyl-2-oxy)-phenoxy]-propionsäure-(trimethylsilyl)-methylesters, das R-Enantiomere des 2-[4-(3,5-Dichlorpyridyl-2-oxy)-phenoxy]-propionsäure-(2-benzyloxy)-ethylesters, 2,4-Dichlorphenoxyessigsäure, 2-(2,4-Dichlorphenoxy)-propionsäure, 4-Chlor-2-methyl-phenoxyessigsäure, 2-(2-Methyl-4-chlor-phenoxy)-propionsäure, 3,5-Diiod-4-hydroxy-benzonitril, 3,5-Dibrom-4-hydroxy-benzonitril sowie Diphenylether und Phenylpyridazine, wie z. B. Pyridate. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größerem Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

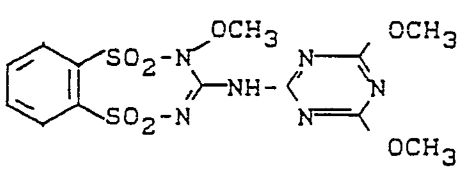

7,1 g (0,025 Mol) Benzol-1,2-disulfonsäurechlorid werden portionsweise bei — 10 °C zu einer Mischung von 5,6 g (0,025 Mol) N'-(4,6-Dimethoxy-1,3,5-triazin-2-yl)-N"-methoxy-guanidin, 6 g (0,025 Mol) DABCO und 60 ml Methylenchlorid gegeben. Man rührt 15 Stunden bei 0 bis — 5 °C nach. Dann wird das Reaktionsgemisch mit eisgekühlter verdünnter Salzsäure und Eiswasser gewaschen. Die Methylenchloridlösung wird getrocknet und eingeengt.

Nach dem Andestillieren erhält man 4,6 g (43 % der Theorie) der Verbindung der oben angegebenen Strukturformel in Form einer amorphen Masse.

Die Substanz wird durch das $H^1$-NMR-Spektrum charakterisiert.

Nach dem im vorausgehenden Beispiel exemplarisch beschriebenen Verfahren können die in der nachstehenden Tabelle 1 aufgeführten Verbindungen der Formel (I) hergestellt werden :

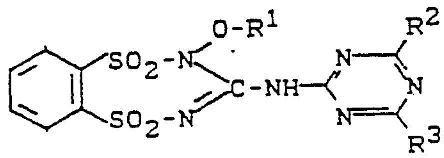

(I)

Tabelle 1

| Beisp.-Nr. | $R^1$ | $R^2$ | $R^3$ | Schmelzpunkt [°C] |
|---|---|---|---|---|
| 2 | $-C_2H_5$ | $-OCH_3$ | $-OCH_3$ | |
| 3 | $-CH_2-CH=CH_2$ | $-OCH_3$ | $-OCH_3$ | |
| 4 | $-CH_3$ | $-OCH_3$ | $-OC_2H_5$ | |
| 5 | $-C_4H_9-n$ | $-OCH_3$ | $-OC_2H_5$ | |
| 6 | $-CH_3$ | $-OCH_3$ | $-N(C_2H_5)_2$ | |
| 7 | $-C_3H_7-n$ | $-SCH_3$ | $-NHC_2H_5$ | |
| 8 | $-CH_3$ | $-CH_3$ | $-OCH_3$ | amorph |
| 9 | $-C_8H_{17}-n$ | $-CH_3$ | $-OCH_3$ | |
| 10 | $-CH_2-$ 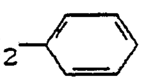 | $-CH_3$ | $-OCH_3$ | |
| 11 | $-CH_3$ | $-OCH_3$ | $-SCH_3$ | |
| 12 | $-CH_3$ | $-CH_3$ | $-OC_2H_5$ | |
| 13 | $-CH_2-$ 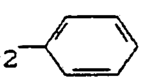 | $-CH_3$ | $-OC_2H_5$ | amorph |
| 14 | $-CH_3$ | $-SCH_3$ | $-NHC_2H_5$ | |
| 15 | $-CH_3$ | $-C_2H_5$ | $-OCH_3$ | |
| 16 | $-CH_3$ | $-CH_3$ | $-CH_3$ | amorph |
| 17 | $-CH_2-CH=CH_2$ | $-CH_3$ | $-CH_3$ | |
| 18 | $-CH_2-$ 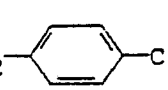 $-COOC_2H_5$ | $-CH_3$ | $-CH_3$ | 195 |

Tabelle 1 (Fortsetzung)

| Beisp.- Nr. | $R^1$ | $R^2$ | $R^3$ | Schmelz- punkt [° C] |
|---|---|---|---|---|
| 19 | $-CH(CH_3)_2$ | $-CH_3$ | $-OCH_3$ | amorph |
| 20 | $-CH(CH_3)_2$ | $-CH_3$ | $-OC_2H_5$ | amorph |
| 21 | $-(CH_2)_7-CH_3$ | $-OCH_3$ | $-OCH_3$ | 110 |
| 22 | $-CH_2$ 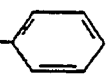 | $-OCH_3$ | $-OCH_3$ | 185 |
| 23 | $-CH_2$ 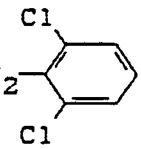 | $-CH_3$ | $-CH_3$ | 150 |

Herstellung von Ausgangsverbindungen der Formel (III)

Beispiel (III-1)

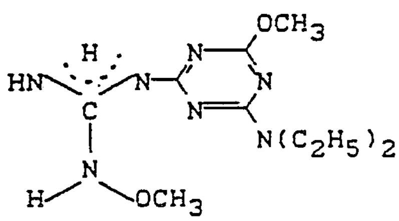

Eine Mischung aus 66,5 g (0,3 Mol) 2-Cyanamino-4-diethylamino-6-methoxy-s-triazin, 50 g (0,8 Mol) O-Methyl-hydroxylamin-Hydrochlorid und 300 ml Ethanol wird 15 Stunden unter Rückfluß zum Sieden erhitzt. Dann wird filtriert, das Filtrat eingeengt, der Rückstand in ca. 200 ml Wasser aufgenommen, mit Natronlauge alkalisch gestellt und das hierbei kristallin anfallende Produkt durch Absaugen isoliert.

Man erhält 35,0 g (43 % der Theorie) N'-(4-Diethylamino-6-methoxy-s-triazin-2-yl)-N''-methoxy-guanidin vom Schmelzpunkt 112 °C.

Analog können die in der nachstehenden Tabelle 2 aufgeführten Verbindungen der Formel (III) hergestellt werden :

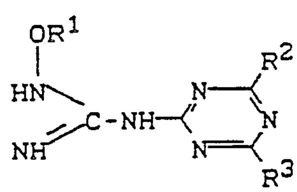

(III)

Tabelle 2

| Beisp. Nr. | $R^1$ | $R^2$ | $R^3$ | Schmelz- punkt [° C] |
|---|---|---|---|---|
| III-2 | $-CH_3$ | $-SCH_3$ | $-NHC_2H_5$ | 117 |
| III-3 | $-CH_3$ | $-SCH_3$ | $-NHCH_3$ | |

Tabelle 2 (Fortsetzung)

| Beisp.-Nr. | $R^1$ | | $R^2$ | $R^3$ | Schmelzpunkt (°C) |
|---|---|---|---|---|---|
| III-4 | $-CH_3$ | | $-Cl$ | $-N(C_2H_5)_2$ | |
| III-5 | $-C_2H_5$ | | $-OCH_3$ | $-N(C_2H_5)_2$ | |
| III-6 | $-C_3H_7-n$ | | $-SCH_3$ | $-NHC_2H_5$ | |
| III-7 | $-CH(CH_3)_2$ | | $-OCH_3$ | $-NHCH_3$ | |
| III-8 | $-CH_2-CH=CH_2$ | | $-OCH_3$ | $-N(CH_3)_2$ | |
| III-9 | $-C_4H_9-n$ | | $-OCH_3$ | $-N(CH_3)_2$ | |
| III-10 | $-CH_2-$ | 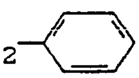 | $-OCH_3$ | $-N(CH_3)_2$ | |
| III-11 | $-CH_2COOC_2H_5$ | | $-OCH_3$ | $-N(CH_3)_2$ | |
| III-12 | $-CH_2-$ | 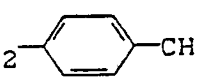 $-CH_3$ | $-OCH_3$ | $-N(CH_3)_2$ | |
| III-13 | $-CH_3$ | | $-CH_3$ | $-OCH_3$ | 126 |
| III-14 | $-CH_2-$ | 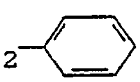 | $-CH_3$ | $-OCH_3$ | 112 |
| III-15 | $-C_8H_{17}$ | | $-CH_3$ | $-OCH_3$ | 95 |
| III-16 | $-CH_2-$ | 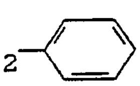 | $-SCH_3$ | $-NHC_2H_5$ | 122 |
| III-17 | $-CH_3$ | | $-CH_3$ | $-OC_2H_5$ | 107 |
| III-18 | $-CH_2-$ | 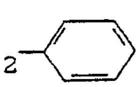 | $-CH_3$ | $-OC_2H_5$ | $n_D^{21} = 1.5824$ |
| III-19 | $-CH_3$ | | $-CH_3$ | $-CH_3$ | 112 |

Tabelle 2 (Fortsetzung)

| Beisp.-Nr. | $R^1$ | $R^2$ | $R^3$ | Schmelzpunkt (°C) |
|---|---|---|---|---|
| III-20 | $-CH_2$ 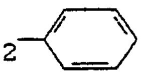 | $-CH_3$ | $-CH_3$ | |
| III-21 | $-CH_2-CH=CH_2$ | $-CH_3$ | $-CH_3$ | |
| III-22 | $-CH_3$ | $-OCH_3$ | $-OCH_3$ | 107 |
| III-23 | $-CH_2$ 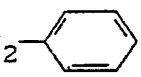 | $-OCH_3$ | $-OCH_3$ | 127 |
| III-24 | $-CH_2-C(CH_3)_3$ | $-CH_3$ | $-CH_3$ | |
| III-25 | $-CH_2CH_2-OCH_3$ | $-OCH_3$ | $-OCH_3$ | |
| III-26 | $-CH_2CH_2-OC_2H_5$ | $-CH_3$ | $-OCH_3$ | |
| III-27 | $-CH_2CH_2-SCH_3$ | $-CH_3$ | $-OC_2H_5$ | |
| III-28 | $-CH_2CH_2-SC_2H_5$ | $-CH_3$ | $-CH_3$ | |
| III-29 | $-CH_2-CONH_2$ | $-OCH_3$ | $-OCH_3$ | |
| III-30 | $-CH_2-CH=CHCl$ | $-CH_3$ | $-OCH_3$ | |
| III-31 | -CH 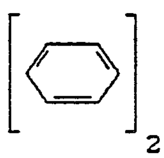 | $-CH_3$ | $-CH_3$ | |
| III-32 | -C 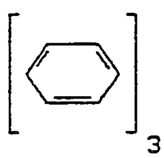 | $-CH_3$ | $-CH_3$ | |
| III-33 | $-CH_3$ | $-C_2H_5$ | $-OCH_3$ | |
| III-34 | $-CH_3$ | $-SCH_3$ | $-OCH_3$ | |
| III-35 | $-CH_3$ | $-OC_2H_5$ | $-OCH_3$ | |

Tabelle 2 (Fortsetzung)

| Beisp.-Nr. | $R^1$ | $R^2$ | $R^3$ | Schmelzpunkt (°C) |
|---|---|---|---|---|
| III-36 | $-CH_3$ | $-NH-C_2H_5$ | $-OCH_3$ | |
| III-37 | $-CH_3$ | $-N(CH_3)_2$ | $-OCH_3$ | |
| III-38 | $-CH_3$ | $-CH_3$ | $-SCH_3$ | |
| III-39 | $-CH_3$ | $-NH-CH_3$ | $-CH_3$ | 128 |
| III-40 | $-CH_3$ | $-N(CH_3)_2$ | $-CH_3$ | 113 |
| III-41 | $-C_3H_7$-i | $-OCH_3$ | $-OCH_3$ | |
| III-42 | $-C_8H_{17}$-n | $-OCH_3$ | $-OCH_3$ | 98 |
| III-43 | $-CH_3$ | $-OCH_3$ | $-NHCH_3$ | 176 |
| III-44 | $-CH_3$ | $-OC_2H_5$ | $-NHCH_3$ | 159 |
| III-45 | $-C_3H_7$-i | $-OC_2H_5$ | $-NHCH_3$ | 152 |
| III-46 | $-CH_2-$ 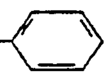 | $-OC_2H_5$ | $-NHCH_3$ | 158 |
| III-47 | $-CH(CH_3)_2$ | $-CH_3$ | $-OCH_3$ | amorph |
| III-48 | $-CH_2CH(CH_3)_2$ | $-CH_3$ | $-OCH_3$ | amorph |
| III-49 | $-CH(CH_3)_2$ | $-CH_3$ | $-OC_2H_5$ | amorph |
| III-50 | $-CH_2CH(CH_3)_2$ | $-CH_3$ | $-OC_2H_5$ | amorph |
| III-51 | $-CH(CH_3)_2$ | $-CH_3$ | $-N(CH_3)_2$ | 100 |
| III-52 | $-CH_2CH(CH_3)_2$ | $-CH_3$ | $-N(CH_3)_2$ | amorph |
| III-53 | $-CH(CH_3)_2$ | $-CH_3$ | $-NHCH_3$ | 127 |
| III-54 | $-CH_2CH(CH_3)_2$ | $-CH_3$ | $-NHCH_3$ | 133 |
| III-55 | $-CH_3$ | $-NHCH_3$ | $-NHCH_3$ | 204 |
| III-56 | $-CH(CH_3)_2$ | $-SCH_3$ | $-NHC_2H_5$ | amorph |
| III-57 | $-CH_2-$ 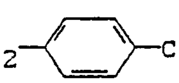 $-COOC_2H_5$ | $-CH_3$ | $-CH_3$ | 133 |

Herstellung von Ausgangsstoffen der Formel (IV)

Beispiel (IV-1)

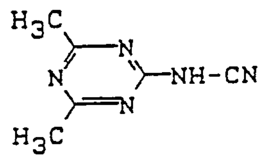

(Verfahren ($a^1$))

Zu einer Suspension von 28,7 g (0,2 Mol) 2-Chlor-4,6-dimethyl-1,3,5-triazin in 100 ml Eiswasser wird unter starkem Rühren in 4 Stunden eine Lösung von 18,0 g (0,21 Mol) Cyanamiddinatriumsalz in 100 ml Wasser bei einer Temperatur von 0 °C bis 5 °C getropft. Anschließend wird bei 20 °C nachgerührt und 15 bis 16 Stunden stehengelassen. Nach Ansäuern mit konz. Salzsäure auf pH = 2 wird abgesaugt, viermal mit 20 ml Eiswasser gewaschen und getrocknet.

Man erhält 19,7 g (66 % der Theorie) 2-Cyanamino-4,6-dimethyl-1,3,5-triazin vom Schmelzpunkt 241 °C (Zers.).

Beispiel (IV-2)

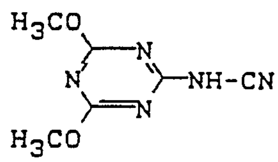

(Verfahren ($a^2$))

Zu einer Suspension von 6 g (0,032 Mol) 4-Chlor-2-cyanamino-6-methoxy-1,3,5-triazin in 50 ml Methanol tropft man bei 20 °C bis 30 °C eine Lösung von 1,6 g (0,069 Mol) Natrium in 20 ml Methanol. Anschließend wird bei 20 °C 15 Stunden nachgerührt. Das Lösungsmittel wird abdestilliert, der Rückstand in 30 ml Wasser gelöst und mit Salzsäure angesäuert. Die entstandenen Kristalle werden abgesaugt und getrocknet.

Man erhält 5,9 g (92 % der Theorie) 2-Cyanamino-4,6-dimethoxy-1,3,5-triazin. Das Produkt wird durch $^1$H-NMR-Spektren charakterisiert.

Analog Beispiel (IV-1) und (IV-2) können die folgenden Verbindungen der Formel (IV) hergestellt werden :

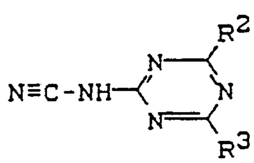

(IV)

Tabelle 3

| Beisp.-Nr. | $R^2$ | $R^3$ | Schmelzpunkt (°C) |
|---|---|---|---|
| IV-3 | $OCH_3$ | $N(C_2H_5)_2$ | 114 |
| IV-4 | $SCH_3$ | $NHC_2H_5$ | |
| IV-5 | $OCH_3$ | $NHCH_3$ | 210 |
| IV-6 | $OC_2H_5$ | $OC_2H_5$ | |
| IV-7 | $OCH_3$ | $CH_3$ | 184 |

Tabelle 3 (Fortsetzung)

| Beisp.-Nr. | $R^2$ | $R^3$ | Schmelzpunkt (°C) |
|---|---|---|---|
| IV-8 | $OCH(CH_3)_2$ | $CH_3$ | |
| IV-9 | $SCH_3$ | $CH_3$ | |
| IV-10 | $SC_2H_5$ | $CH_3$ | |
| IV-11 | $SCH(CH_3)_2$ | $CH_3$ | |
| IV-12 | $NHCH_3$ | $CH_3$ | |
| IV-13 | $NHCH(CH_3)_2$ | $CH_3$ | |
| IV-14 | $N(CH_3)_2$ | $CH_3$ | 267 |
| IV-15 | $OC_2H_5$ | $CH_3$ | 195 (Zers.) |
| IV-16 | $C_2H_5$ | $OCH_3$ | |
| IV-17 | $SCH_3$ | $OCH_3$ | |
| IV-18 | $OC_2H_5$ | $OCH_3$ | |
| IV-19 | $OCH_3$ | $NHCH_3$ | |
| IV-20 | $OCH_3$ | $N(CH_3)_2$ | >260 |
| IV-21 | $N(CH_3)_2$ | $N(CH_3)_2$ | >260 |
| IV-22 | $NHC_3H_7$ | $NHC_3H_7$ | 264 |
| IV-23 | $NHCH(CH_3)_2$ | $NHCH(CH_3)_2$ | 259 |
| IV-24 | $NHC_2H_5$ | $NHC_2H_5$ | 248 |
| IV-25 | $OCH_3$ | $NHC_2H_5$ | 266 |
| IV-26 | $OCH_3$ | $NHCH(CH_3)_2$ | 193 |
| IV-27 | $NHC_4H_9$ | $NHC_4H_9$ | 260 |
| IV-28 | $SCH_3$ | $SCH_3$ | 206 |
| IV-29 | $OC_2H_5$ | $NHCH_3$ | 227 |
| IV-30 | $NHCH_3$ | $NHCH_3$ | >260 |
| IV-31 | $OC_2H_5$ | $NHC_2H_5$ | 184 |
| IV-32 | $NH_2$ | $CH_3$ | >260 |
| IV-33 | $NHCH_3$ | $CH_3$ | 276 |
| IV-34 | $NHC_2H_5$ | $CH_3$ | 238 |

Herstellung von Ausgangsstoffen der Formel (IVa)

Beispiel (IVa-1)

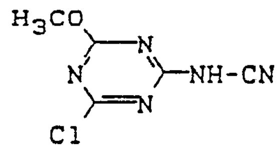

Eine Lösung von 9 g (0,043 Mol) 4-Chlor-2-cyanamino-6-methoxy-1,3,5-triazin-Natriumsalz in 90 ml Wasser wird mit Salzsäure angesäuert und die entstandenen Kristalle werden anschließend abgesaugt.

Man erhält 6,7 g (84 % der Theorie) 4-Chlor-2-cyanamino-6-methoxy-1,3,5-triazin vom Schmelzpunkt > 260 °C. Das Produkt wird durch $^1$H-NMR-Spektren charakterisiert.

Analog Beispiel (IVa-1) können die folgenden Verbindungen der Formel (IVa) hergestellt werden.

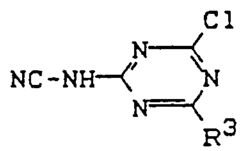

(IVa)

Tabelle 4

| Beisp.-Nr. | $R^3$ | Schmelzpunkt (°C) |
|---|---|---|
| IVa-2 | $CH_3$ | 250 (Zers.) |
| IVa-3 | Cl | >250 |
| IVa-4 | $N(C_2H_5)_2$ | 156 |
| IVa-5 | $NHCH_3$ | >260 |
| IVa-6 | $NHC_4H_9$ | 158 |
| IVa-7 | $NHC_3H_7$ | 136 |
| IVa-8 | $NHCH(CH_3)_2$ | 167 |
| IVa-9 | $NHC_2H_5$ | 153 |
| IVa-10 | $N(CH_3)_2$ | 222 |

Beispiel A

Pre-emergence-Test

Lösungsmittel : 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dahei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit. Dies gilt insbesondere für die Verbindung gemäß Herstellungsbeispiel 1, welche z. B. bei einer Aufwandmenge von 0,125 kg/ha selektiv in Weizen ist, wobei die Unkrautwirkung gegen Chenopodium, Matricaria, Sinapis, Xanthium, Agropyron und Alopecurus bei 80-100 % liegt.

Beispiel B

Post-emergence-Test

Lösungsmittel : 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe vopn 5-15 cm haben so, daß die jeweils gewünschten Wirkstoffmengen pro Flächeneinheit ausgebracht werden. Die Konzentration der Spritzbrühe wird so gewählt, daß in 2 000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit. Insbesondere gilt dies für die Verbindung gemäß Herstellungsbeispiel 1, welche z. B. bei einer Aufwandmenge von 0,5 kg/ha selektiv in Gerste und Weisen ist, wobei die Unkrautwirkung gegen Abutilon, Galium, Sinapis, Stellaria, Alopecurus und Echinochloa bei 80-100 % liegt.

## Patentansprüche

1. Benzo-[b]-1,4,5,7-dithiadiazepin-1,1,4,4-tetraoxid-Derivate (Benzodisultame) der allgemeinen Formel (I)

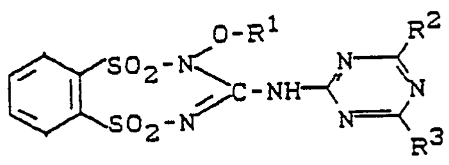

(I)

in welcher

$R^1$ für $C_1$-$C_{12}$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxycarbonyl, $C_1$-$C_4$-Alkylamino-carbonyl oder Di-($C_1$-$C_4$-alkyl)-amino-carbonyl substituiert ist], für $C_3$-$C_6$-Alkenyl [welches gegebenenfalls durch Fluor, Chlor oder Brom substituiert ist], $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxycarbonyl substituiert ist] oder für Phenyl [welches gegebenenfalls durch Fluor, Chlor, Nitro, Cyano $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist] steht,

$R^2$ für Wasserstoff, Fluor, Chlor, Hydroxy, Cyclopropyl, Methyl [welches gegebenenfalls durch Fluor

und/oder Chlor substituiert ist], für $C_1$-$C_3$-Alkoxy [welches gegebenenfalls durch Fluor und/oder Chlor substituiert ist], für $C_1$-$C_3$-Alkylthio [welches gegebenenfalls durch Fluor und/oder Chlor substituiert ist], Amino, für $C_1$-$C_3$-Alkyl- oder Di-($C_1$-$C_3$-alkyl)- amino [welche gegebenenfalls durch Fluor substituiert sind] steht und

$R^3$ für Wasserstoff, Fluor, Chlor, Hydroxy, Cyclopropyl, Methyl [welches gegebenenfalls durch Fluor und/oder Chlor substituiert ist], für $C_1$-$C_3$-Alkoxy [welches gegebenenfalls durch Fluor und/oder Chlor substituiert ist], für $C_1$-$C_3$-Alkylthio [welches gegebenenfalls durch Fluor und/oder Chlor substituiert ist], Amino, für $C_1$-$C_3$-Alkyl- oder Di-($C_1$-$C_3$-alkyl)-amino [welche gegebenenfalls durch Fluor substituiert sind] steht.

2. Verbindungen der allgemeinen Formel (I) gemäß Anspruch 1, dadurch gekennzeichnet, daß darin

$R^1$ für $C_1$-$C_8$-Alkyl [welches gegebenenfalls durch Fluor oder Chlor substituiert ist], $C_3$-$C_4$-Alkenyl, $C_1$-$C_2$-Alkoxy-carbonylmethyl, Phenyl, Phenylethyl oder Benzyl [welches gegebenenfalls durch Fluor, Chlor, Nitro, Cyano, Methyl, Methoxy oder Methoxy-carbonyl substituiert ist] steht,

$R^2$ für Wasserstoff, Chlor, Methyl, Methoxy, Ethoxy, Methylthio, Ethylthio, Methylamino, Ethylamino, Dimethylamino oder Diethylamino steht und

$R^3$ für Wasserstoff, Chlor, Methyl, Methoxy, Ethoxy, Methylthio, Ethylthio, Methylamino, Ethylamino, Dimethylamino oder Diethylamino steht.

3. Verfahren zur Herstellung von Benzodisultamen der allgemeinen Formel (I) gemäß Anspruch 1, dadurch gekennzeichnet, daß man Benzol-1,2-disulfonsäuredichlorid der Formel (II)

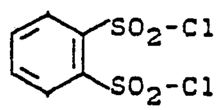

(II)

mit Oxyguanidin-Derivaten der Formel (III)

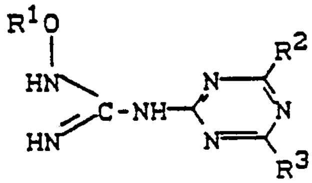

(III)

in welcher

$R^1$, $R^2$ und $R^3$ die oben angegebenen Bedeutungen haben,

in Gegenwart von Säureakzeptoren und gegebenenfalls in Gegenwart von Verdünnungsmitteln umsetzt.

4. Herbizide Mittel, gekennzeichnet durch einen Gehalt an mindestens einem Benzodisultam der allgemeinen Formel (I) gemäß Anspruch 1.

5. Verwendung von Benzodisultamen der allgemeinen Formel (I) gemäß Anspruch 1 zur Bekämpfung von unerwünschtem Pflanzenwachstum.

6. Verfahren zur Herstellung von herbiziden Mitteln, dadurch gekennzeichnet, daß man Benzodisultame der allgemeinen Formel (I) gemäß Anspruch 1 mit Streckmitteln und/oder oberflächenaktiven Mitteln vermischt.

**Claims**

1. Benzo-[b]-1,4,5,7-dithiadiazepine-1,1,4,4-tetraoxide derivatives (benzodisultams) of the general formula (I)

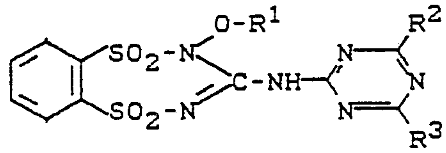

(I)

in which

$R^1$ represents $C_1$-$C_{12}$-alkyl [which is optionally substituted by fluorine, chlorine, cyano, $C_1$-$C_4$-alkoxy, $C_1$-$C_4$-alkylthio, $C_1$-$C_4$-alkylsulphinyl, $C_1$-$C_4$-alkylsulphonyl, $C_1$-$C_4$-alkyl-carbonyl, $C_1$-$C_4$-alkoxy-carbonyl, $C_1$-$C_4$-alkylamino-carbonyl or di-($C_1$-$C_4$-alkyl)-amino-carbonyl], $C_3$-$C_6$-alkenyl [which is optionally substituted by fluorine, chlorine or bromine], $C_3$-$C_6$-alkinyl, $C_3$-$C_6$-cycloalkyl, $C_3$-$C_6$-cycloalkyl-$C_1$-$C_2$-alkyl, phenyl-$C_1$-$C_2$-alkyl [which is optionally substituted by fluorine, chlorine, nitro, cyano,

$C_1$-$C_4$-alkyl, $C_1$-$C_4$-alkoxy or $C_1$-$C_4$-alkoxycarbonyl] or phenyl [which is optionally substituted by fluorine, chlorine, nitro, cyano, $C_1$-$C_4$-alkyl, trifluoromethyl, $C_1$-$C_4$-alkoxy, $C_1$-$C_2$-fluoroalkoxy, $C_1$-$C_4$-alkylthio, trifluoromethylthio or $C_1$-$C_4$-alkoxy-carbonyl],

$R^2$ represents hydrogen, fluorine, chlorine, hydroxyl, cyclopropyl, methyl [which is optionally substituted by fluorine and/or chlorine], $C_1$-$C_3$-alkoxy [which is optionally substituted by fluorine and/or chlorine], $C_1$-$C_3$-alkylthio [which is optionally substituted by fluorine and/or chlorine], amino, $C_1$-$C_3$-alkyl- or di-($C_1$-$C_3$-alkyl)-amino [which is optionally substituted by fluorine] and

$R^3$ represents hydrogen, fluorine, chlorine, hydroxyl, cyclopropyl, methyl [which is optionally substituted by fluorine and/or chlorine], $C_1$-$C_3$-alkoxy [which is optionally substituted by fluorine and/or chlorine], $C_1$-$C_3$-alkylthio [which is optionally substituted by fluorine and/or chlorine], amino, or $C_1$-$C_3$-alkyl- or di-($C_1$-$C_3$-alkyl)-amino [which is optionally substituted by fluorine].

2. Compounds of the general formula (I) according to Claim 1, characterized in that, in this formula,

$R^1$ represents $C_1$-$C_8$-alkyl [which is optionally substituted by fluorine or chlorine], $C_3$-$C_4$-alkenyl, $C_1$-$C_2$-alkoxy-carbonyl-methyl, phenyl, phenylethyl or benzyl [which is optionally substituted by fluorine, chlorine, nitro, cyano, methyl, methoxy or methoxy-carbonyl],

$R^2$ represents hydrogen, chlorine, methyl, methoxy, ethoxy, methylthio, ethylthio, methylamino, ethylamino, dimethylamino or diethylamino and

$R^3$ represents hydrogen, chlorine, methyl, methoxy, ethoxy, methylthio, ethylthio, methylamino, ethylamino, dimethylamino or diethylamino.

3. Process for the preparation of benzodisultams of the general formula (I) according to Claim 1, characterized in that benzene-1,2-disulphonyl dichloride of the formula (II)

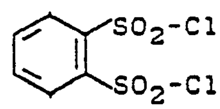

(II) ,

is reacted with oxyguanidine derivatives of the formula (III)

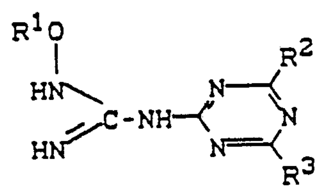

(III)

in which

$R^1$, $R^2$ and $R^3$ have the meanings given above, in the presence of acid acceptors and, if appropriate, in the presence of diluents.

4. Herbicidal agents, characterized in that they contain at least one benzodisultam of the general formula (I) according to Claim 1.

5. Use of benzodisultams of the general formula (I) according to Claim 1, for combating undesired plant growth.

6. Process for the preparation of herbicidal agents, characterized in that benzodisultams of the general formula (I) according to Claim 1 are mixed with extenders and/or surface-active agents.

**Revendications**

1. Dérivés de 1,1,4,4-tétroxyde de benzo-[b]-1,4,5,7-dithiadiazépine (benzodisultames), de formule générale (I)

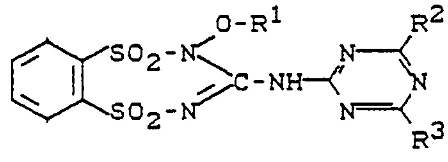

(I)

dans laquelle

$R^1$ est un groupe alkyle en $C_1$ à $C_{12}$ [qui est substitué, le cas échéant, par du fluor, du chlore, un radical cyano, alkoxy en $C_1$ à $C_4$, alkylthio en $C_1$ à $C_4$, alkylsulfinyle en $C_1$ à $C_4$, alkylsulfonyle en $C_1$ à $C_4$, (alkyle en $C_1$ à $C_4$)-carbonyle, (alkoxy en $C_1$ à $C_4$)-carbonyle, (alkyle en $C_1$ à $C_4$)-aminocarbonyle ou di-(alkyle en $C_1$ à $C_4$)-aminocarbonyle], un groupe alcényle en $C_3$ à $C_6$ [qui est substitué, le cas échéant, par

du fluor, du chlore ou du brome], un groupe alcynyle en $C_3$ à $C_6$, cycloalkyle en $C_3$ à $C_6$, (cycloalkyle en $C_3$ à $C_6$)-(alkyle en $C_1$ ou $C_2$), phényl-(alkyle en $C_1$ ou $C_2$) [qui est substitué, le cas échéant, par du fluor, du chlore, un radical nitro, cyano, alkyle en $C_1$ à $C_4$, alkoxy en $C_1$ à $C_4$ ou (alkoxy en $C_1$ à $C_4$)-carbonyle] ou un groupe phényle [qui est substitué, le cas échéant, par du fluor, du chlore, un radical nitro, cyano, alkyle en $C_1$ à $C_4$, trifluorométhyle, alkoxy en $C_1$ à $C_4$, fluoralkoxy en $C_1$ ou $C_2$, alkylthio en $C_1$ à $C_4$, trifluorométhylthio ou (alkoxy en $C_1$ à $C_4$)-carbonyle], dans laquelle en outre

$R^2$ représente l'hydrogène, le fluor, le chlore, un groupe hydroxy, cyclopropyle, méthyle [qui est substitué, le cas échéant, par du fluor et/ou du chlore], un groupe alkoxy en $C_1$ à $C_3$ [qui est substitué, le cas échéant, par du fluor et/ou du chlore], un groupe alkylthio en $C_1$ à $C_3$ [qui est substitué, le cas échéant, par du fluor et/ou du chlore], un groupe amino, un groupe (alkyle en $C_1$ à $C_3$)-amino ou un groupe di-(alkyle en $C_1$ à $C_3$)-amino [qui sont substitués, le cas échéant, par du fluor] et

$R^3$ est de l'hydrogène, du fluor, du chlore, un groupe hydroxy, cyclopropyle, méthyle [qui est substitué, le cas échéant, par du fluor et/ou du chlore], un groupe alkoxy en $C_1$ à $C_3$ [qui est substitué, le cas échéant, par du fluor et/ou du chlore], un groupe alkylthio en $C_1$ à $C_3$ [qui est substitué, le cas échéant, par du fluor et/ou du chlore], un groupe amino, un groupe (alkyle en $C_1$ à $C_3$)-amino ou un groupe di-(alkyle en $C_1$ à $C_3$)-amino [qui sont substitués, le cas échéant, par du fluor].

2. Composés de formule générale (I) suivant la revendication 1, caractérisés en ce que, dans cette formule

$R^1$ est un groupe alkyle en $C_1$ à $C_8$ [qui est substitué, le cas échéant, par du fluor ou du chlore], un groupe alcényle en $C_3$ ou $C_4$, (alkoxy en $C_1$ ou $C_2$)-carbonylméthyle, phényle, phényléthyle ou benzyle [qui est substitué, le cas échéant, par du fluor, du chlore, un radical nitro, cyano, méthyle, méthoxy ou méthoxycarbonyle],

$R^2$ représente de l'hydrogène, du chlore, un groupe méthyle, méthoxy, éthoxy, méthylthio, éthylthio, méthylamino, éthylamino, diméthylamino ou diéthylamino et

$R^3$ représente de l'hydrogène, du chlore, un groupe méthyle, méthoxy, éthoxy, méthylthio, éthylthio, méthylamino, éthylamino, diméthylamino ou diéthylamino.

3. Procédé de production de benzodisultames de formule générale (I) suivant la revendication 1, caractérisé en ce qu'on fait réagir le dichlorure d'acide benzène-1,2-disulfonique de formule (II)

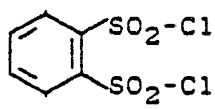

(II)

avec des dérivés d'oxyguanidine de formule (III)

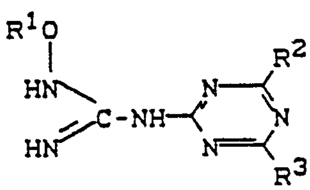

(III)

dans laquelle

$R^1$, $R^2$ et $R^3$ ont les définitions indiquées ci-dessus, en présence d'accepteurs d'acides et, le cas échéant, en présence de diluants.

4. Compositions herbicides, caractérisées par une teneur en au moins un benzodisultame de formule générale (I) suivant la revendication 1.

5. Utilisation de benzodisultames de formule générale (I) suivant la revendication 1 pour combattre une végétation non désirée.

6. Procédé de préparation de compositions herbicides, caractérisé en ce qu'on mélange des benzodisultames de formule générale (I) suivant la revendication 1 avec des diluants et/ou des agents tensio-actifs.